# EUROPEAN PATENT APPLICATION

(11) **EP 1 925 330 A2**
(43) Date of publication of application: **28.05.2008**
(21) Application number: 07254466.1
(22) Date of filing: 15.11.2007
(51) Int. Cl.: A61M 16/04

(54) **Suction apparatus and connectors**

(30) Priority: 25.11.2006 GB 0623535
(71) Applicant: Smiths Group plc, London, NW11 8DS (GB)
(72) Inventor: Field, Stephen James, Canterbury Kent CT4 5 LA (GB)
(74) Representative: Flint, Jonathan McNeill

(57) **Abstract**

A tracheal tube 1 has a sealing cuff 4 towards its patient end 2 and a suction aperture 8 just above the cuff. The suction aperture 8 connects via a lumen 7 with a suction line 9 terminated by a connector 10. One end of the connector 10 is tapered and has a port 20 for connection to a suction source. The connector 10 also has a side port 17 in which the nose 31 of a syringe 32 can be inserted to inject a lavage fluid out of the suction aperture 8 to help loosen secretions. When not in use, the side port 17 is closed by a valve 140 or 240 or by a closure cap 21 with formations 24 and 25 for sealing the two ports 20 and 17.

## Description

This invention relates to suction apparatus of the kind including a medico-surgical device having a suction aperture and a suction line connected at one end in communication with the suction aperture, the suction line having a connector at its opposite end, the connector having a first port opening into a bore extending along the suction line and adapted to be connected with a source of suction.

There are various medical procedures where suctioning is needed, such as to remove accumulations of secretions, irrigation fluid, blood or other body fluids. One example is cuffed tracheal tubes where it is desirable to remove secretions that collect in the trachea above the cuff. With such tracheal tubes, a suction lumen may be extruded within the wall of the tube and open via an aperture towards its patient end just above the cuff. The suction lumen is connected, towards its other end, to a small-bore suction line terminated with a coupling to which a source of suction can be connected. An example of a tracheal tube having such a suction lumen is described in US5201310. In some cases it is necessary to introduce a lavage solution, such as saline, via the suction lumen to aid in the removal of the secretions. This is presently done by disconnecting the machine end coupling on the suction line from the suction circuit and instead connecting a syringe containing the lavage solution to the coupling. It is, however, undesirable to have to break the suction circuit each time that lavage needs to be administered since this takes time, can result in the unhygenic release of secretions from the suction tube and can increase the risk of infection. There are various other medical devices where a suction line is used to administer other fluids as well as providing a path for suctioning.

It is an object of the present invention to provide alternative suction apparatus and connectors.

According to one aspect of the present invention there is provided suction apparatus of the above-specified kind, characterised in that the connector has a second port also opening into the bore along the suction line and adapted to be connected with a source of fluid for supply to the suction aperture, and that the connector has a closure for closing the second port when not in use.

The medico-surgical device may be a tube, such as a tracheal tube. The fluid may be a lavage fluid and the source may be provided by a syringe having a nose arranged for insertion into the-second port. The closure may be a valve or a removable closure cap. The cap may be tethered with the connector and may have respective formations for closing the first and second ports.

According to another aspect of the present invention there is provided a connector for suction apparatus according to the above one aspect of the present invention.

Suction apparatus in the form of an endotracheal tube according to the present invention will now be described, by way of example, with reference to the accompanying drawings, in which:
- Figure 1: is a side elevation view of tube;
- Figure 2: is a perspective view of the machine end of the suction line and connector with its cap open, to an enlarged scale;
- Figure 3: is a side elevation view of the connector with a suction line and lavage syringe attached;
- Figure 4: is a cross-sectional side elevation view of the connector with its cap closed;
- Figure 5: is a perspective view of an alternative connector with a lavage syringe connected;
- Figure 6: is a cross-sectional view of the connector of Figure 5 without the syringe and with the suction port closed; and
- Figure 7: is a cross-sectional view of another alternative connector.

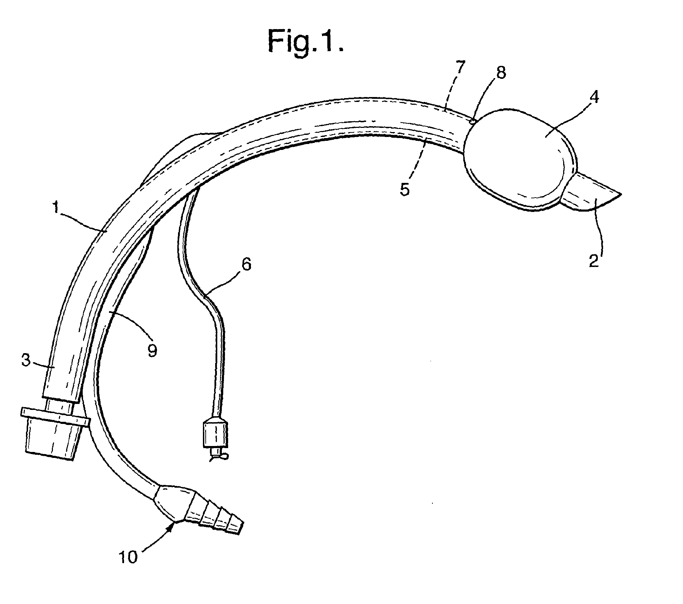

With reference first to Figures 1 to 4 the endotracheal tube includes a curved shaft 1 with an angled patient end 2 and a machine end 3 at the opposite end of the shaft. An inflatable sealing cuff 4 is attached to the shaft 1 close to its patient end 2 and this is inflated or deflated via an inflation lumen 5 extruded within the wall of the shaft along the inside curve of the shaft. A small-bore inflation line 6 is attached to an opening in the inflation lumen 5 about midway along the tube. The tube also has a suction/lavage lumen 7 extruded within the wall of the shaft 1 and extending along the outside curve of the shaft. The suction/lavage lumen 7 opens through an aperture 8 formed in the outside of the shaft 1 close to the upper, machine end of the cuff 4. The suction/lavage lumen 7 is connected with one end of a small-bore suction/lavage line 9 about midway along the length of the tube. The suction/lavage line 9 is a flexible tube and is terminated at its other, free end by a novel coupling or connector 10.

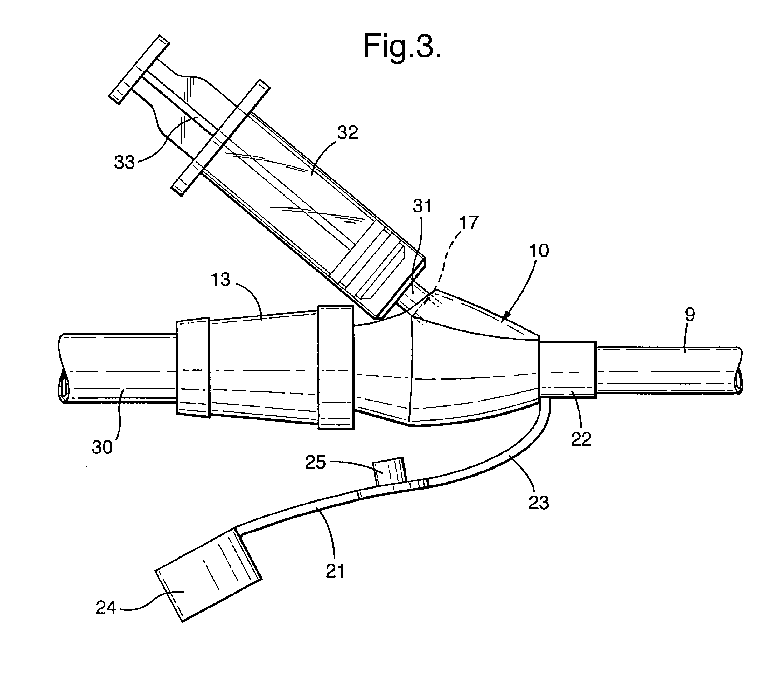

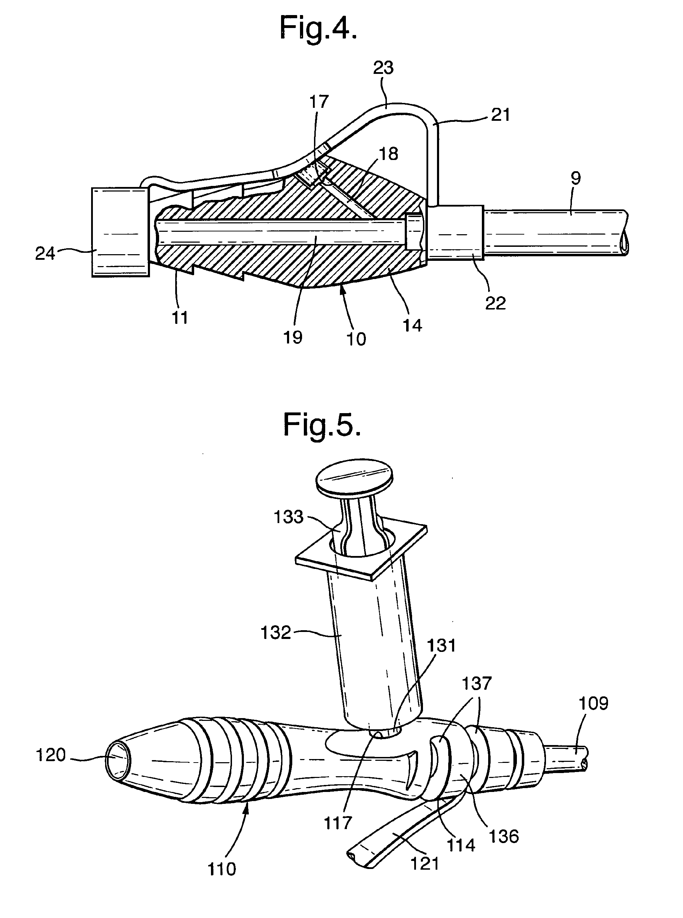

The connector 10 is moulded from a relatively hard plastics material about the end of the line 9. The connector 10 has a machine end portion 11, which is circular in section and is formed with several tapered teeth 12 increasing in diameter from the machine to the patient end. These teeth serve to provide a grip for a resilient tubing connector 13 (Fig 3) pushed onto the end of the connector. The patient end portion 14 of the connector 10 is substantially smooth on its outer surface and has a wedge-shape protrusion 15 on one side providing a flat face 16 inclined at an angle of about 45° to the connector axis and facing towards the machine end. The face 16 has a circular aperture 17 providing a port opening into a side bore 18 (Fig 4), which communicates with an axial bore 19 extending along the connector 10 from the suction line 9 to the machine end 11 of the connector where it opens via a suction port 20.

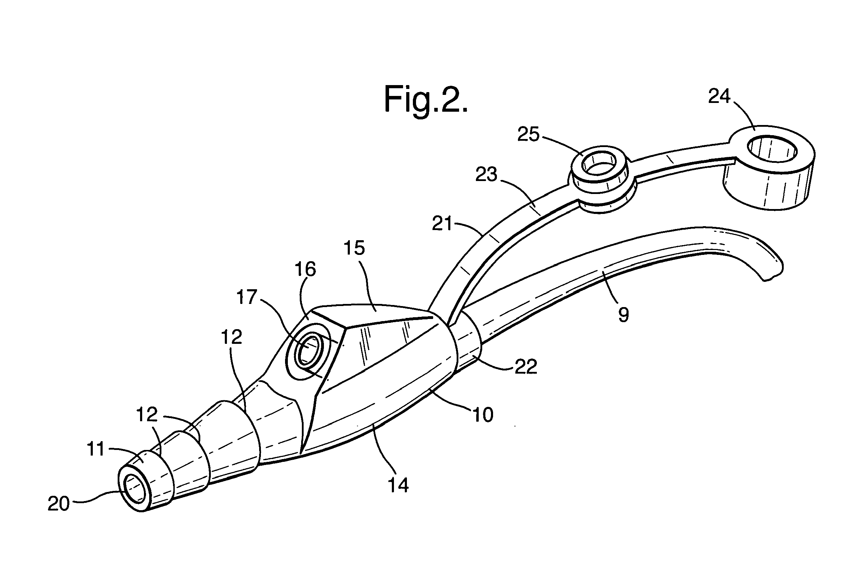

The connector 10 also includes a closure in the form of a two-point moulded plastics cap 21 having a collar 22 at one end rotatably attached to the line 9 where it meets the connector. A flexible tether 23 extends from the collar 22 and is moulded with a female closure 24 at its opposite end adapted to fit over the machine end 11 of the connector 10 and seal the suction port 20. The tether 23 also has a male closure 25 about midway along its length and adapted to fit sealingly within the second port 17. Figure 2 shows the cap 21 disconnected so that both ports 17 and 20 are open. Figure 4 shows the cap 21 secured to close both ports 17 and 20.

In normal use, for suctioning, a main suction line 30 is connected to the machine end 11 of the connector 10 and the second port 17 is closed by the cap 21 so that there was no loss of suction pressure via the lavage port. Secretions that collect above the cuff 4 are removed via the aperture 8, the lumen 7, the suction line 9, the connector 10 and the main suction line 30. When it is necessary to supply lavage fluid, such as saline solution, to the trachea, the suction is turned off but the main suction line 30 is left connected to the connector 10. The cap 21 is pulled off the second port 17 and the nose 31 of a syringe 32 containing the lavage fluid is pushed into sealing engagement in the lavage port 17. The plunger 33 of the syringe 32 is depressed to dispense the fluid from the syringe into the line 9 via the connector 10, and hence into the space between the tube and the trachea above the cuff 4. Suction can then be turned on to aspirate the secretions and other fluid above the cuff 4. The syringe 32 may be left connected and a further dose of lavage fluid administered if need be or the syringe could be uncoupled from the connector 10 and the aperture 17 closed again with the cap 21.

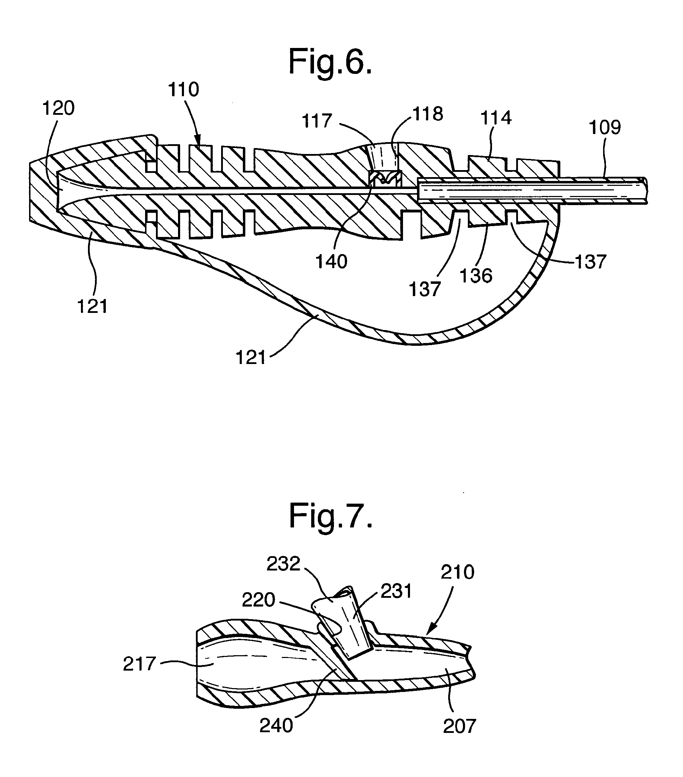

Instead of using a cap to seal the lavage port, it would be possible to use a valve, as in the modified connector shown in Figures 5 and 6. Equivalent features to those in the connector shown in Figures 1 to 4 have been given the same reference numerals with the addition of 100. The connector 110 is a single piece moulding with an integral cap 121. The patient end 114 of the connector 110 is overmoulded onto a short length at the rear of the suction/lavage line 109 and this overmoulded patient end portion is formed with annular ribs 136 and grooves 137 to make it more flexible than the main part of the connector and thereby act as a strain relief feature to prevent an abrupt bend or kink in the suction lavage line should it be bent to one side close to the connector. The lavage port 117 is directed radially outwards and the side bore 118 includes a simple one-way valve 140, such as a duck-bill valve, that is normally closed but is opened by increased fluid pressure as the plunger 133 of the syringe 132 is depressed. This connector 110 has a single closure cap 121 for closing the suction port 120 when the connector is disconnected from the suction source. It will be appreciated, however, that, as with the connector shown in Figures 1 to 4, the connector can be left connected to the suction source during lavage.

When the suction source is turned off for lavage, the resistance to flow towards the suction source will be slightly higher than the resistance to flow towards the open suction aperture so the lavage liquid will flow in this direction. This is the case both with the connector shown in Figures 5 and 6 and that shown in Figures 1 to 4. If desired, the main suction line 30 could be clamped during lavage to reduce further flow towards the suction source.

Various other valves could be used that enable fluid to be supplied to the connector but prevent escape of suction pressure when the syringe or other fluid source is not connected. For example, the valve could be of the kind that is depressed and opened by contact with the syringe nose or other coupling and closes when the syringe is removed.

The connector 210 shown in Figure 7 is arranged so that the suction port 217 is automatically closed by insertion of the nose 231 of a syringe 232. In this particular arrangement, the connector 210 has a flap valve 240 that is pushed down by the nose 231 of the syringe 232 when it is inserted in the lavage port 220 so that the flap valve occludes the passage between the suction port 217 and the suction/lavage lumen 207.

Alternatively, the connector could incorporate a three-way stop-cock to enable the suction/lavage lumen to be connected to the lavage source or the suction source.

The connector of the present invention provides a very compact, inexpensive arrangement to facilitate lavage or the supply of other fluids and avoids the need to disconnect the suction line or to use additional lavage lines. Although the invention is particularly suited for suctioning and lavage above the cuff of an endotracheal or tracheostomy tube, it will be appreciated that it could have application in other medical devices where suctioning and intermittent lavage needs to be carried out. The invention is not confined to use with lavage fluids but could be used to introduce other fluids, such as antibacterial liquids, analgesics or anaesthetic fluids, lubricants or the like. The source of fluid need not be a syringe but could be of various other kinds, such as, for example a gravity feed from a suspended fluid bag via tubing and a connector.

## Claims

1. Suction apparatus including a medico-surgical device (1) having a suction aperture (8) and a suction line (7, 9) connected at one end in communication with the suction aperture, the suction line (7, 9) having a connector (10) at its opposite end, the connector (10) having a first port (20) opening into a bore extending along the suction line (7, 9) and adapted to be connected with a source of suction, **characterised in that** the connector (10) has a second port (17) also opening into the bore along the suction line and adapted to be connected with a source of fluid (32) for supply to the suction aperture (8), and that the connector (10) has a closure (21, 140, 240) for closing the second port (17) when not in use.

2. Suction apparatus according to Claim 1, **characterised in that** the medico-surgical device is a tube (1).

3. Suction apparatus according to Claim 2, **characterised in that** the tube is a tracheal tube (1).

4. Suction apparatus according to any one of the preceding claims, **characterised in that** the fluid is a lavage fluid.

5. Suction apparatus according to any one of the preceding claims, **characterised in that** the source of fluid is provided by a syringe (32) having a nose (31) arranged for insertion into the second port (17).

6. Suction apparatus according to any one of the preceding claims, **characterised in that** the closure is a valve (140, 240).

7. Suction apparatus according to any one of Claims 1 to 5, **characterised in that** the closure is a removable closure cap (21).

8. Suction apparatus according to Claim 7, **characterised in that** the cap (21) is tethered with the connector (10).

9. Suction apparatus according to Claim 7 or 8, **characterised in that** the cap (21) has respective formations (24 and 25) for closing the first and second ports (20 and 17)..

10. A connector (10) for apparatus according to any one of the preceding claims.
